# EUROPEAN PATENT APPLICATION

(11) **EP 2 272 803 A2**
(43) Date of publication of application: **12.01.2011**
(21) Application number: 10163189.3
(22) Date of filing: 19.05.2010
(51) Int. Cl.: C02F 1/461

(54) **Electrolysis apparatus and device comprising the same**

(30) Priority: 26.06.2009 KR 20090057547
(71) Applicant: SAMSUNG ELECTRONICS CO., LTD., Suwon-si, Gyeonggi-do (KR)
(72) Inventor: Kim, Jee Yong, Seoul (KR)
(74) Representative: Grünecker, Kinkeldey, Stockmair & Schwanhäusser Anwaltssozietät

(57) **Abstract**

An electrolyte apparatus wherein an electric-field inducing material capable of forming a uniform electric field between electrodes is arranged between the electrodes, so as to obtain sufficient electrolysis, although a low voltage is applied thereto, based on the fact that a voltage applied to electrodes is closely related to an electric field generated therebetween, and a device including the same is provided. The electric-field inducing material includes a conductive material bound to a non-conductive material, wherein the conductive material is randomly distributed in the accepting area.

## Description

### BACKGROUND

### 1. Field

Exemplary embodiments relate to an electrolysis apparatus for electrolyzing fluid by applying a low voltage to electrodes, and a device comprising the same.

### 2. Description of the Related Art

Electrolysis apparatuses are applied to devices such as air-conditioners, refrigerators and washing machines so as to impart additional functions to purify or sterilize air or water to the devices.

Such an electrolysis apparatus electrolyzes fluids such as water or air passing through an electric field generated between electrodes. In recent years, electrolysis apparatuses remove or deodorize contaminants such as dust or particles contained in water or air using plasma generated by high-voltage discharge which is induced by applying a high voltage to electrodes.

In the case where a low voltage is applied between electrodes, when water contains no electrolyte or, if present, contains an extremely small amount, electrolysis does not occur. For this reason, electrolysis apparatuses require an electric power source to supply several kV of high voltage electricity, take a long time for sufficient electrolysis and thus exhibit increased power consumption.

In addition, when a high voltage is applied to electrodes to electrolyze highly insulating fluid such as air, dielectric breakdown occurs, thus generating harmful byproducts such as ozone or nitric oxide (NOx) and negatively affecting the human body and environment.

In an attempt to solve these problems, a great deal of research has been conducted into techniques for adhering dielectric barrier discharge (DBD) films into electrodes to generate plasma under atmospheric pressure or utilizing a plasma-catalyst hybrid process. However, DBD prevents generation of flame or activates various chemical reactions under atmospheric pressure at low temperatures, but requires application of high voltages to electrodes. In addition, the plasma catalyst hybrid process improves absorption force owing to use of metal catalysts such as titanium or platinum, but requires application of considerably high voltages to electrodes.

### SUMMARY

In accordance with an aspect of exemplary embodiments, there is provided an electrolyte apparatus for electrolyzing a fluid by inducing formation of a uniform electric field between electrodes, although a low voltage is applied thereto, and a device comprising the same.

In accordance with an aspect of exemplary embodiments, there is provided an electrolyte apparatus for electrolyzing a fluid using a low voltage to prevent generation of harmful byproducts, and a device comprising the same.

In accordance with an aspect of exemplary embodiments, there is provided an electrolysis device including: a plurality of electrodes spaced from one another, to provide an accepting area, through which a fluid passes; a power source supplier to apply electricity to the electrodes; and an electric-field inducing material to mediate transfer of electrons in the accepting area and induce formation of a uniform electric field.

The electric-field inducing material may be in the form of a fiber, a foam, a particle, a bead or a pellet.

The electric-field inducing material may include a conductive material bound to a non-conductive material, wherein the conductive material is randomly distributed in the accepting area.

The conductive material may be bound to the non-conductive material by coating.

The coating may be carried out by dipping a metal powder in a non-conductive thread, followed by roll-molding, or using chemical vapor deposition (CVD).

The conductive material may be bound to the non-conductive material by coupling.

The conductive material may be bound to the non-conductive material by mix-spinning.

The conductive material may be a conductor or a semiconductor.

The conductor may be Ag, Cu, Au, Al, Ca or Mg.

The non-conductive material may be nylon, polypropylene (PP) or polyethylene terephthalate (PET).

The electric-field inducing material may include an electrolyte with electrical conductivity adsorbed on a paper to mediate transfer of electrons, and the electrolysis device may further include a water bath containing an electrolyte solution in which the paper is dipped.

In accordance with an aspect of exemplary embodiments, there is provided an electrolysis device including: a positively-charged first electrode; a negatively-charged second electrode which faces the first electrode; a non-conductive material filled between the first electrode and the second electrode; and a conductive material bound to the non-conductive material, to mediate transfer of electrons from the second electrode to the first electrode.

The electrolysis device may further include: a conductive material randomly distributed in the accepting area to form a uniform electric field between the first electrode and the second electrode.

The conductive material may be adhered to the surface or inside of the non-conductive material.

In accordance with an aspect of exemplary embodiments, there is provided a device for electrolyzing a fluid using an electrolysis device, wherein the electrolysis device includes: a plurality of electrodes, corresponding to a cathode and an anode; and an electric-field inducing material to mediate transfer of electrons between the electrodes and thus induce formation of a uniform electric field therebetween, wherein the electrolysis device is mounted in a passage, allowing a fluid to be supplied into the device for electrolyzing the fluid.

The electrolysis device may be provided in a refrigerator to electrolyze tap water supplied from an external source and provide potable water.

### BRIEF DESCRIPTION OF THE DRAWINGS

These and/or other aspects will become apparent and more readily appreciated from the following description of exemplary embodiments, taken in conjunction with the accompanying drawings of which:
FIG. 1 is a schematic view illustrating an electrolysis apparatus according to an exemplary embodiment;
FIG. 2 is a view illustrating the configuration of an electrolysis apparatus according to an exemplary embodiment;
FIG. 3 is an enlarged sectional view of FIG. 2A;
FIG. 4 is a view illustrating a mechanism wherein formation of an electric field is induced in the electrolysis apparatus shown in FIG. 2;
FIG. 5 is a view illustrating the configuration of an electrolysis apparatus according to an exemplary embodiment;
FIG. 6 is an enlarged sectional view of FIG. 5B.
FIG. 7 is a view illustrating the configuration of an electrolysis apparatus according to another exemplary embodiment;
FIG. 8 is a view illustrating a mechanism wherein formation of an electric field is induced in the electrolysis apparatus shown in FIG. 7; and
FIG. 9 is a view illustrating a refrigerator to which the electrolysis apparatus according to an exemplary embodiment is applied.

### DETAILED DESCRIPTION

Reference will now be made in detail to exemplary embodiments, examples of which are illustrated in the accompanying drawings, wherein like reference numerals refer to like elements throughout.

In accordance with an electrolysis apparatus of an exemplary embodiment, based on the fact that a voltage applied to electrodes is closely related to an electric field between the electrodes, an electric-field inducing material capable of forming a uniform electric field between the electrodes is arranged therebetween, so as to obtain sufficient electrolysis, although a low voltage is applied thereto. The electric-field inducing material used herein includes a conductive material to mediate movement of electrons.

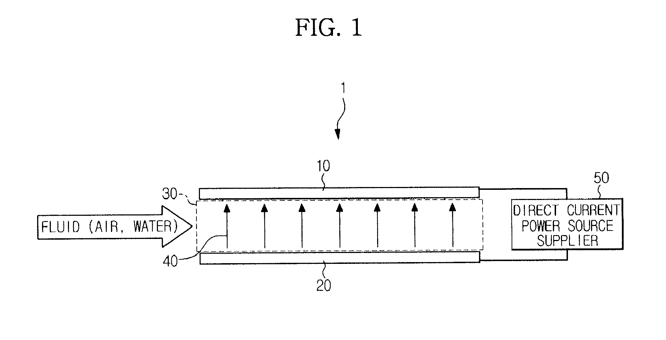

FIG. 1 is a schematic view illustrating an electrolysis apparatus 1 according to an exemplary embodiment.

A first electrode 10 and a second electrode 20 are arranged such that they face each other and are spaced from each other by a predetermined distance. An electric-field inducing material is filled in an accepting area, through which fluid such as water or air passes, provided between the first and second electrodes 10 and 20.

The first electrode 10 and the second electrode 20 may be realized in the form of a plate or mesh. One of these electrodes is designated by a cathode and the other is designated by an anode. For convenience, the first electrode 10 and the second electrode 20 are referred to as a cathode and an anode, respectively. Although not illustrated in this particular exemplary embodiment, one of the electrodes may receive a direct current power source and be ground to the other, when an electric field is generated between both electrodes.

A direct current power source supplier 50 is electrically connected to these electrodes 10 and 20, to supply a direct current power source thereto. The direct current power source supplier 50 applies a low direct current power source not higher than 100V thereto. In an exemplary embodiment, a direct current power source is applied to both electrodes for electrolysis and is not limited thereto. Alternatively, an alternating current power source may be applied to both electrodes to perform electrolysis.

When a low voltage direct current power source is applied to the electrodes 10 and 20, electrons are discharged from the negatively-charged (-) electrode 20 and then move to the positively-charged (+) electrode 10 to form an electric field 40. In the formation of the electric field, the electric-field inducing material 30 filled between the electrodes serves to mediate transfer of electrons. In the case where an electric field is excessively unbalanced, that is, when a strong electric field is formed in a portion of both electrodes which face each other, while a weak electric field is formed in the remaining region, a higher voltage should be applied so as to compensate for the disparity therebetween.

As such, an electric field should be leveled between electrodes facing each other so as to reduce an application voltage. Accordingly, in various exemplary embodiments illustrated below, constitutions and operations to cause the electric-field inducing material arranged between electric fields to induce uniform formation of electric field are illustrated in detail.

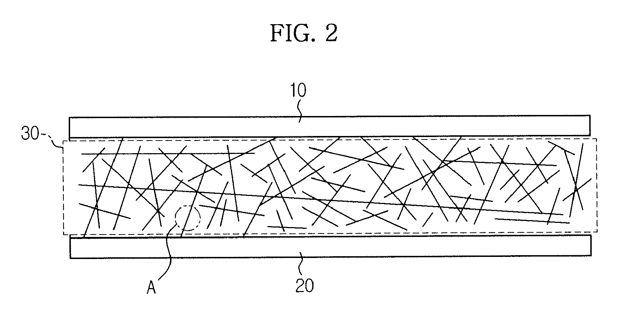

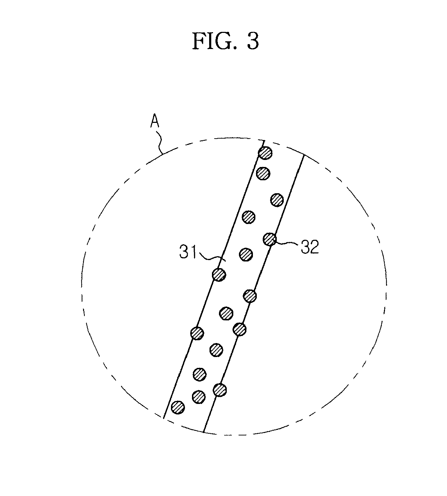

FIG. 2 is a view illustrating the structure of an electrolysis apparatus according to an exemplary embodiment, and FIG. 3 is an enlarged sectional view of FIG. 2A. Like elements mentioned hereinbefore are designated by like reference numerals. The direct current power source supplier 50 may be applied to supply a direct current power source to the both electrodes 10 and 20.

The electric field-inducing material 30 includes a conductive material 32 to mediate transfer of electrons and the conductive material 32 mounted to a supporter 31 is dispersed in an accepting area.

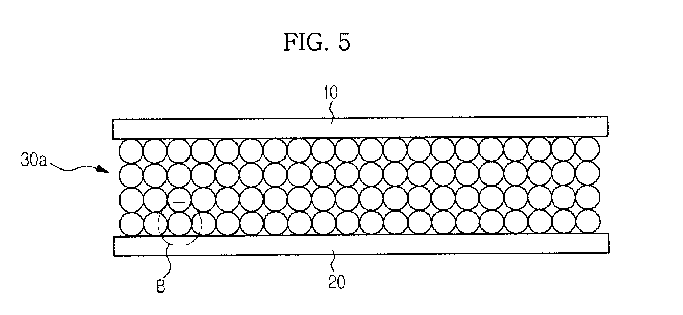

As shown in FIG. 2, the supporter 31 may be in the form of tangled fibers but is not limited thereto. The supporter 31 may be in the form of a foam or particle, when mounted to the conductive material 32. In addition, in an exemplary embodiment shown in FIG. 5, the supporter 31 may have a cyclic shape.

The supporter 31 is a non-conductive material exhibiting low electrical conductivity and examples thereof include insulating materials such as nylon, polypropylene (PP) and polyethylene terephthalate (PET). When the supporter 31 exhibits high electrical conductivity, both electrodes receive an electric current, thus preventing electrical discharge.

The conductive material 32 may be a conductor or semiconductor and examples thereof include highly-conductive metals such as Ag, Cu, Au, Al, Ca and Mg.

As shown in FIG. 3, the conductive material 32 should be bound to the supporter 31, to allow the conductive material 32 to be present not exclusively, but on the surface or inside of the supporter 31. The binding of the conductive material 32 to the supporter 31 is carried out using coating, coupling, wherein molding is realized by coordinating a metal element to an organic material, and mix spinning wherein molding is realized by mixing a non-conductive material with a conductive material and spinning the mixture in an electric field. The coating may be carried out by dipping a metal powder made of a mixture of various metals having high electrical conductivity together with non-conductive threads in a given solution for a predetermined time, followed by roll-molding. Another coating method is chemical vapor deposition (CVD) wherein molding is realized by vaporizing various metals having high electrical conductivity and spraying the same to a non-conductive material.

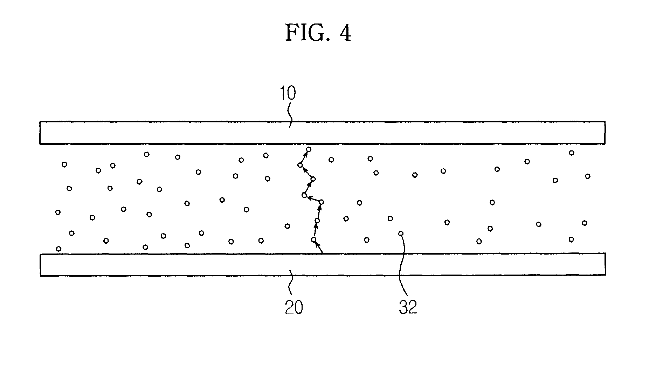

The conductive material 32 bound to the supporter 31, as shown in FIG. 4, serves as a medium to mediate transfer of electrons discharged from the negatively-charged (-) electrode 20. The conductive material 32 is randomly dispersed between both electrodes 10 and 20, thus enabling uniform dispersion of the electric field generated during application of a direct current power source thereto. The conductive material 32 contributes to inhibiting electric field disparity, although it cannot allow the identical electric field to be formed in all positions of electrodes facing each other. This uniform formation of electric field enables sufficient electrolysis of fluids passing through an area provided between electrodes.

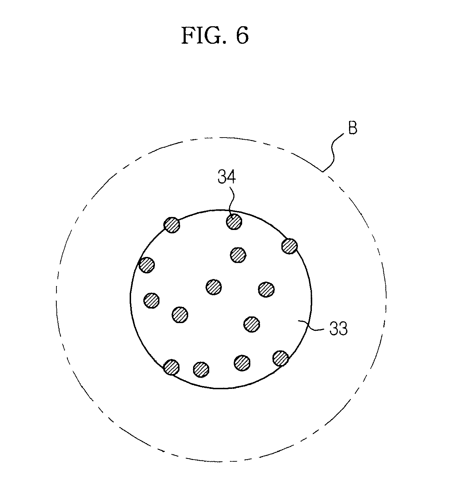

FIG. 5 is a view illustrating the structure of an electrolysis apparatus according to another exemplary embodiment, and FIG. 6 is an enlarged sectional view of FIG. 5B. Like elements mentioned hereinbefore are designated by like reference numerals. The direct current power source supplier 50 may be applied to supply a direct current power source to both electrodes 10 and 20.

The electrolysis apparatus of an exemplary embodiment of Figure 5 is similar to that of an exemplary embodiment mentioned hereinbefore, and these two exemplary embodiments have the same configuration wherein an electric-field inducing material 30a containing a conductive material to mediate transfer of electrons is filled between the electrodes. Only structure of the electric-field inducing material 30a is different.

The supporter 33 used herein is a non-conductive insulating material and the conductive material 32 may be a conductor or semiconductor, which is the same as in an exemplary embodiment as mentioned hereinbefore.

The electric-field inducing material 30a has a structure wherein the conductive material 34 is present on the surface of the cyclic supporter 33 or inside the same. The cyclic shape of the supporter 33 may be realized in the form of a bead or pellet.

The conductive material 34 bound to the supporter 33 serves as a medium to mediate transfer of electrons discharged from the negatively-charged (-) electrode 20. The conductive material 32 is randomly dispersed between both electrodes 10 and 20, thus enabling uniform dispersion of the electric field generated during application of a direct current power source thereto. The conductive material 32 contributes to inhibiting electric field disparity, although it cannot allow the identical electric field to be formed in all positions of electrodes facing each other. This uniform formation of electric field enables sufficient electrolysis of fluids passing through an area provided between electrodes.

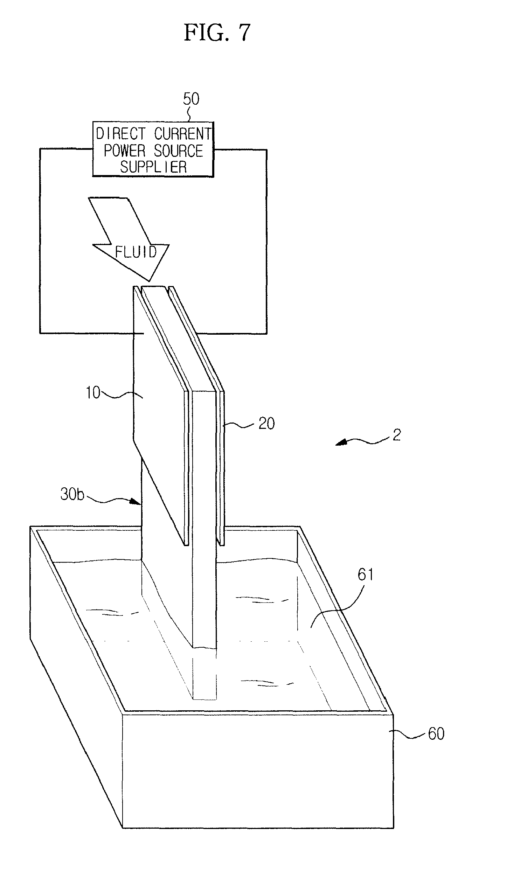

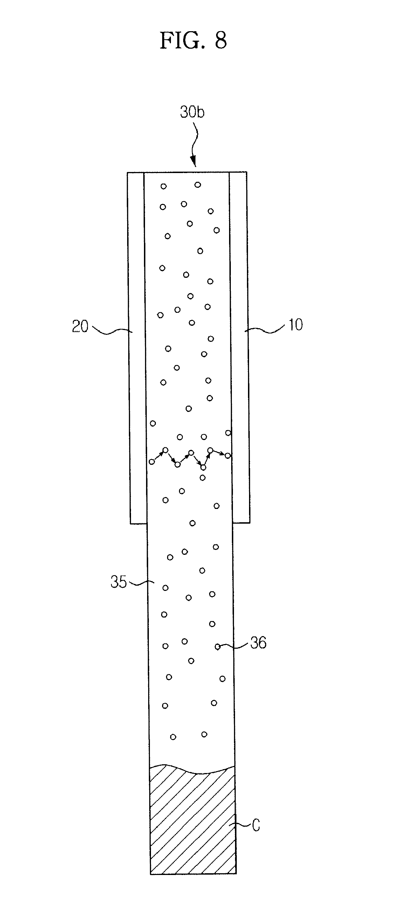

FIG. 7 is a view illustrating the structure of an electrolysis apparatus according to an exemplary embodiment, and FIG. 8 is a view illustrating a mechanism wherein formation of an electric field is induced in an electrolysis apparatus shown in FIG. 7. In FIGs. 7 and 8, the two electrodes 10 and 20 and the direct current power source supplier 50 of an exemplary embodiment shown in Figures 7 and 8 perform the same functions as in exemplary embodiments mentioned hereinbefore.

As shown in FIG. 7, a fluid moves parallel to the two electrodes, which is only given for illustration. That is, the two electrodes may be in the form of a mesh shape, allowing the fluid to pass through one of the electrodes and then be discharged through the opposing electrode. When the fluid passes between the electrodes, electrolysis occurs.

The electrolysis apparatus 2 according to an exemplary embodiment as shown in FIG. 7 is different from those of exemplary embodiments mentioned hereinbefore in view of the structure of an electric-field inducing material 30b filled between the two electrodes 10 and 20.

The electric-field inducing material 30b includes a highly-adherent supporter 35 as a non-conductive material. The supporter 35 extends longitudinally to form a plate shape. In an exemplary embodiment, the supporter 35 utilizes a pasteboard, but is not limited thereto. The supporter 35 may be a non-conductive material exhibiting superior water-adsorption.

The end of the supporter 35 closely contacts an area provided between the two electrodes 10 and 20 and the other is dipped in an electrolyte solution 61 filled in a water bath 60. The two electrodes 10 and 20 are spaced from the chamber 60 to prevent electricity from being conducted therebetween.

The electrolyte solution 61 may be tap water containing sodium ions such as an electrolyte with high electrical conductivity. Pure water processed by a purifier is unsuitable for use.

Referring to FIG. 8, a portion C of the electric-field inducing material 30b is dipped in the electrolyte solution 61 contained in the water bath 60. With passage of time, a highly electrically conductive electrolyte 36 adsorbed on the dipped portion C elevates upward and diffuses to the water surface. As such, the electrolyte 36 having high electrical conductivity is present in a position corresponding to the two electrodes 10 and 20. Under this condition, a direct current power source supplier 50 applies a direct current power source to the electrodes, the adsorbed electrolyte 36 mediates transfer of electrons discharged from the negatively-charged (-) electrode 20. The electrolyte 36 is randomly dispersed between the two electrodes 10 and 20, thus enabling uniform dispersion of the electric field generated during application of a direct current power source thereto. This uniform formation of electric field enables sufficient electrolysis of fluids passing through an area provided between electrodes, although a low voltage is applied thereto.

As apparent from the afore-mentioned exemplary embodiments, the electrolysis apparatus 1 or 2 prevents disparity of an electric field and induces uniform distribution thereof using a conductive material or a highly electrically conductive material to mediate transfer of electrons. As a result, a fluid can be electrolyzed, although a low voltage is applied to electrodes, thus shortening electrolysis time and reducing power consumption due to high electrical conductivity. Furthermore, a direct current power source having a voltage lower than conventional cases can be used, thus satisfying superior electrolysis capability, while reducing the thickness of electrode plates and extending the distance between the electrodes.

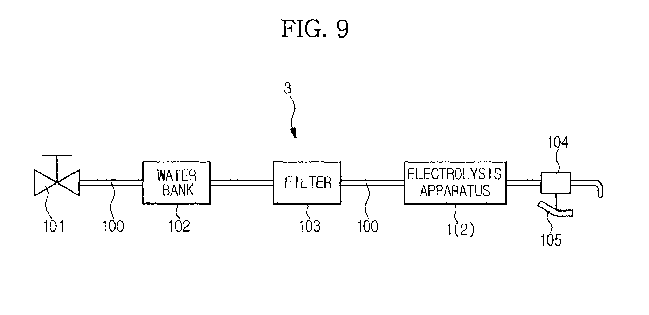

FIG. 9 is a view illustrating a refrigerator to which the electrolysis apparatus according to an exemplary embodiment is applied. This device 3 is provided with an electrolysis apparatus 1 or 2 mounted in a supply passage 100 connected to an external water source 101. A water bank 102 and a filter 103 are provided in an upper part of the electrolysis apparatus 1 or 2.

The tap water supplied from the external water source 101 is stored in a predetermined amount in the water bank 102 and then transferred to the filter 103. The filter 103 filters foreign materials contained in the tap water.

The electrolysis apparatus 1 or 2 performs electrolysis on tap water wherein foreign materials are removed through the filter 103 by applying a low voltage to the two electrodes 10 and 20. In the process of electrolysis, minute contaminants contained in tap water are removed and sterilized and harmful odors are removed to provide potable water. As a result, a valve 104 opens to provide potable water, when a user operates a switch 105.

Furthermore, the electrolysis apparatus 1 or 2 according to exemplary embodiments may be utilized in a variety of applications. For example, the electrolysis apparatus 1 or 2 can deodorize or sterilize an inner area or a heat-exchange device mounted in refrigerators and air-conditioning systems, or deodorize or sterilize laundries provided in washing machines.

Although a few exemplary embodiments have been shown and described, it would be appreciated by those skilled in the art that changes may be made in these exemplary embodiments without departing from the principles and spirit of the disclosure, the scope of which is defined in the claims and their equivalents.

## Claims

1. An electrolysis device comprising:
a plurality of electrodes spaced from one another, to provide an accepting area, through which a fluid passes;
a power source supplier to apply electricity to the electrodes;
an electric-field inducing material to mediate transfer of electrons in the accepting area and induce formation of a uniform electric field.

2. The electrolysis device according to claim 1, wherein the electric-field inducing material is in the form of a fiber, a foam, a particle, a bead or a pellet.

3. The electrolysis device according to claim 1, wherein the electric-field inducing material comprises a conductive material bound to a non-conductive material, wherein the conductive material is randomly distributed in the accepting area.

4. The electrolysis device according to claim 3, wherein the conductive material is bound to the non-conductive material by coating,
wherein the coating is carried out by dipping a metal powder in a non-conductive thread, followed by roll-molding, or using chemical vapor deposition (CVD).

5. The electrolysis device according to claim 3, wherein the conductive material is bound to the non-conductive material by coupling.

6. The electrolysis device according to claim 3, wherein the conductive material is bound to the non-conductive material by mix-spinning.

7. The electrolysis device according to claim 3, wherein the conductive material is a conductor or a semiconductor.

8. The electrolysis device according to claim 7, wherein the conductor is Ag, Cu, Au, Al, Ca or Mg.

9. The electrolysis device according to claim 3, wherein the non-conductive material is nylon, polypropylene (PP) or polyethylene terephthalate (PET).

10. The electrolysis device according to claim 1, wherein the electric-field inducing material comprises an electrolyte with electrical conductivity adsorbed on a paper to mediate transfer of electrons, and
the electrolysis device further comprises a water bath containing an electrolyte solution in which the paper is dipped.

11. An electrolysis device comprising:
a positively-charged first electrode;
a negatively-charged second electrode which faces the first electrode;
a non-conductive material filled between the first electrode and the second electrode; and
a conductive material bound to the non-conductive material, to mediate transfer of electrons from the second electrode to the first electrode.

12. The electrolysis device according to claim 11, further comprising:
a conductive material randomly distributed in the accepting area to form a uniform electric field between the first electrode and the second electrode.

13. The electrolysis device according to claim 11, wherein the conductive material is adhered to the surface or inside of the non-conductive material.

14. A device for electrolyzing a fluid using an electrolysis device,
wherein the electrolysis device comprises:
a plurality of electrodes, corresponding to a cathode and an anode; and
an electric-field inducing material to mediate transfer of electrons between the electrodes and induce formation of a uniform electric field therebetween,
wherein the electrolysis device is mounted in a passage, allowing a fluid to be supplied into the device for electrolyzing the fluid.

15. The device according to claim 14, wherein the electrolysis device is provided in a refrigerator to electrolyze tap water supplied from an external source and provide potable water.
